(19) Europäisches Patentamt
European Patent Office
Office européen des brevets

(11) Numéro de publication: **0 218 622 B1**

(12) # FASCICULE DE BREVET EUROPEEN publi e en application de l'article 158, paragraphe 3 de la CB3

(45) Date de publication de fascicule du brevet: **10.07.91**

(51) Int. Cl.5: C07C 233/11, C07C 233/51, C07C 323/59, C07D 233/64, C07D 295/18, A61K 7/42

(21) Numéro de dépôt: **86901914.1**

(22) Date de dépôt: **28.03.86**

(86) Numéro de dépôt internationale : **PCT/FR86/00108**

(87) Numéro de publication internationale : **WO 86/05783 (09.10.86 86/22)**

(54) **AMIDES DE L'ACIDE PARA-METHOXYCINNAMIQUE ET LEURS UTILISATION COMME FILTRES SOLAIRES.**

(30) Priorité: **28.03.85 FR 8504898**

(43) Date de publication de la demande: **22.04.87 Bulletin 87/17**

(45) Mention de la délivrance du brevet: **10.07.91 Bulletin 91/28**

(84) Etats contractants désignés: **AT BE CH DE FR GB IT LI LU NL SE**

(56) Documents cités:
**DE-A- 2 015 447**
**DE-A- 2 932 923**
**FR-A- 2 204 628**
**FR-A- 2 545 355**

**Chemical Abstracts, vol. 59, no. 7, 30 septembre 1963, Columbus, Ohio, (US) voir colonnes 7316, 7317 & JP, A, 18098**

(73) Titulaire: **UNIVERSITE LOUIS PASTEUR (STRASBOURG I)**
**4, rue Blaise Pascal**
**F-67070 Strasbourg Cédex(FR)**

(72) Inventeur: **JUNG, Louis**
**205, route d'Oberhausbergen**
**F-67200 Strasbourg(FR)**
Inventeur: **ROBERT, Dominique**
**248, avenue de la Vaugine**
**F-83300 Draguignan(FR)**

(74) Mandataire: **Ores, Irène et al**
**CABINET ORES 6, Avenue de Messine**
**F-75008 Paris(FR)**

**EP 0 218 622 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Chemical Abstracts, volume 84, no. 22, 31 mai 1976, Columbus, Ohio, (US) voir page 346, no. 155513c & JP,A, 7533986

Chemical Abstracts, vol. 94, no. 9, 2 mars 1981, Columbus, Ohio, (US) L. Ren-Li et al.: "Chemical structure-physiological activity relation in cinnamamides and their analogs. II. Relation between chemical structure and anticonvulsant effect"

Chemical Abstracts, vol. 73, no. 19, 9 novembre 1970, Columbus, Ohio, (US) J.L. Pousset et al.: "Isolation of urocanylglycine, a urinary derivative of histidine"

Dictionary of organic compounds, ed. 5, vol. 4 (1982), page 3714

japan Kokai 7533986

## Description

La présente invention concerne de nouveaux dérivés de l'acide para-méthoxycinnamique, ainsi que l'obtention desdits dérivés. Elle concerne également les compositions dermo-pharmaceutiques et cosmétiques les contenant.

Les dérivés de l'acide para-méthoxycinnamique utilisés actuellement comme filtres solaires et qui sont contenus dans des compositions cosmétologiques à titre d'ingrédient actif répondent à la formule I suivante :

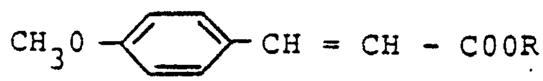

I

R représentant des groupes alkyle pouvant éventuellement supporter des fonctions soufrées (cf Demande de Brevet français n° 84 10009 du 22 juin 1984).

Le composé de formule I le plus connu répond à la formule Ibis ci-après :

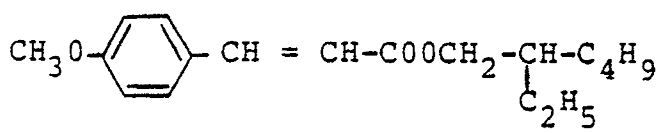

Ibis

Ce composé présente un certain nombre d'inconvénients qui sont ceux de tous les filtres solaires utilisés actuellement :

- faible adhérence au niveau de la peau (élimination lors des bains de mer ou par transpiration)
- passage dans le sang chez l'homme au cours de l'utilisation
- au vu de cette faible adhérence, plusieurs applications sont nécessaires (toutes les 2 à 3 heures), ce qui entraîne à chaque application une nouvelle pénétration dans le sang
- le produit s'hydrolyse sous l'influence des estérases cutanées et plasmatiques en acide p.méthoxycinnamique. Or, ce dernier, au niveau cutané, perd le pouvoir filtrant recherché vis-à-vis des UVB.

La Demande de Brevet français précitée décrit également de nouveaux filtres solaires à structure soufrée et/ou aminoacide, caractérisés en ce qu'ils comportent une fonction ester. Ces composés ont un pouvoir filtrant vis-à-vis des UVB identique ou supérieur à celui des composés de formule I ci-dessus.

Les Inventeurs ont pu, d'autre part, mettre en évidence la fixation au niveau de la surface de l'épiderme de ces nouveaux filtres solaires,contrairement aux composés de formule I, cette mise en évidence ayant été réalisée en utilisant le lapin comme modèle expérimental : en effet, la peau de lapin est nettement plus perméable que celle de l'homme et permet une meilleure quantification du filtre solaire ou de son métabolite dans le sang (méthode de dosage par CPG/S.M. permettant des déterminations quantitatives spécifiques de l'ordre du ng/ml). Pour certaines formulations galéniques de produits de formule I on retrouve dans le sang jusqu'à 10-20 % du filtre solaire appliqué (thèse D. CLAUS, doctorat d'Université, Strasbourg 1982 : figure 1).

Pour les esters soufrés, aucune trace de filtre solaire ou de métabolite n'a été trouvée dans le sang de lapin.

Cependant, les Inventeurs ont pu mettre en évidence qu'une partie de la fonction ester est hydrolysée sous l'influence des estérases cutanées, en utilisant un prélèvement de peau préalablement rasée de la face ventrale d'un rat. L'hypoderme a été soigneusement décollé à l'aide d'une lame de rasoir. L'épiderme et le derme restants ont été coupés en fines lamelles et introduits dans un erlenmeyer contenant 20 ml de tampon phosphate pH 8. Les lamelles ont alors été soumises à l'action d'un Potter à grandes dents afin d'éviter la libération des protéases des noyaux cellulaires. La préparation obtenue a été centrifugée à 25000 t/mn et le surnageant a été prélevé. 10 μM du produit à tester ont été mis en suspension dans 4 ml de l'homogénat. Au bout de 12 h, les solutions ont été analysées par CCM.

Le filtre solaire constitué par un ester soufré est totalement hydrolysé au bout de 12 h. Cependant, il faut noter que le pouvoir estérasique des homogénats de peau (qui n'est pas d'origine microbienne) est beaucoup plus réactif que celui de la peau normale ; pour des cellules entières, les estérases sont surtout localisées à l'intérieur des cellules. Cependant, ce type d'enzyme est libéré au moment de la kératinisation

c'est-à-dire à la surface de la peau (J. WEPIERRE, Labo-Pharma, Probl. Tech. 1983, 31, 730-734).

On connaît dans la littérature, notamment dans le Dictionary of organic compounds 1982, ed. 5, vol. 4 page 37141, l'amide de l'acide p-méthoxycinnamique ; toutefois ce document ne précise pas les propriétés éventuelles de cet amide.

On trouve dans la littérature la description de divers amides de l'acide p-méthoxycinnamique utilisables en tant qu'agents de protection contre les coups de soleil et en particulier dans CHEMICAL ABSTRACTS Vol. 59, N° 7, 30 septembre 1963, la description de composés de formule p-$MeOC_6H_4CH=CHCONX(CH_2)_nY$ dans laquelle X est de l'hydrogène ou un alkyle inférieur, Y est un groupe hydroxy ou acide et n est égal à 1 ou 2 et, de façon spécifique, la description de la p-méthoxycinnamoylsarcosine.

Il est également connu d'utiliser l'acide urocanique de formule II ci-après, comme filtre solaire :

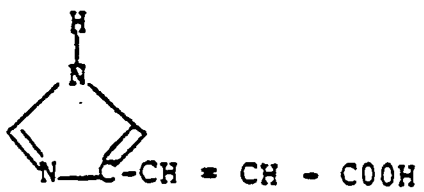

II

On connaît également des amides de l'acide urocanique aptes à absorber la lumière ultra-violette, et notamment le stéarylamide et le dodécylamide de l'acide urocanique (cf CHEMICAL ABSTRACTS Vol. 84, N° 22, 31 mai 1976, page 346, N° 155513c et Demande de Brevet japonais (Japon Kokai 7533986) ainsi que Brevet français AJINOMOTO N° 2 204 628).

Toutefois, dans ces cas, on est également confronté aux problèmes de faible adhérence à la peau et de passage dans le sang de l'homme, exposés plus haut. En effet dans Ces documents, il n'y est fait aucune mention de ce que les amides décrits pourraient ou non constituer un filtre absorbant simultanément ou non les UVA et les UVB ou de ce que qu'ils pourraient ou non pénétrer dans le sang à travers la barrière dermo-épidermique.

La présente invention a en conséquence pour but de pourvoir à des dérivés chromophores de types para-méthoxycinnamique qui répondent mieux aux nécessités de la pratique que les filtres solaires constitués par des dérivés connus d'acide para-méthoxycinnamique notamment en ce que les nouveaux dérivés présentent une stabilité chimique supérieure à celle des dérivés connus en tant que filtres solaires et une plus grande adhérence à la peau lorsqu'ils sont appliqués sur cette dernière, et en ce que ces nouveaux dérivés sont retenus au niveau de l'épiderme et ne passent pas dans le sang, non plus que d'éventuels métabolites de ces dérivés.

La présente invention a pour objet de nouveaux amides de l'acide para-méthoxycinnamique, caractérisés en ce qu'ils répondent à la formule III ci-après :

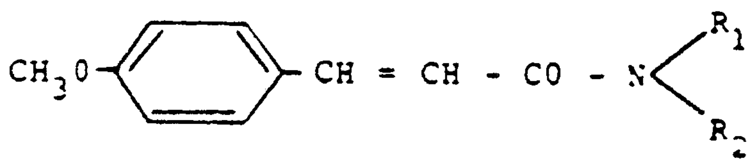

III

dans laquelle :

- les double-liaisons éthyléniques peuvent exister soit sous la forme trans, soit sous la forme cis,
- $R_1$ et $R_2$ peuvent être de l'hydrogène, un groupe alkyle, un groupe aryle, sous réserve que $R_1$ ne soit pas un atome d'hydrogène lorsque $R_2$ est un groupe alkyle, ou un atome d'hydrogène,
- $R_1$ et $R_2$ peuvent former avec l'atome d'azote un groupe cycloalkyle hétérocyclique substitué,ou non substitué autre que la pipéridine,
- $R_1$ ou $R_2$ peuvent aussi être un groupe :
  * de formule

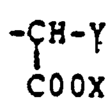 où X et Y

peuvent être un groupe alkyle ou un groupe aryle,
* de formule

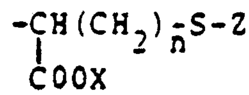

où X peut être un groupe alkyle ou un groupe aryle, Z peut être l'hydrogène, un groupe alkyle, un groupe aryle et où n est un nombre entier compris entre 1 et 6 mais de préférence égal à 1 ou 2,
* de formule

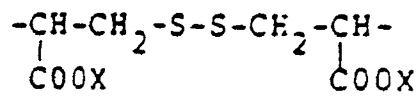

sur lequel sont fixées deux molécules de chromophores filtrantes du type acide para-méthoxycinnamique ou acide urocanique et où X peut être un groupe alkyle ou un groupe aryle,

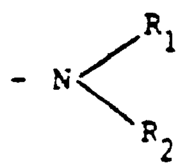

peut être un dérivé de l'acide urocanique de type

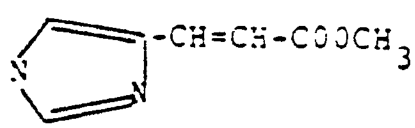

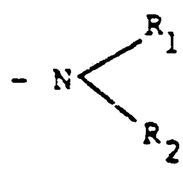

peut être une structure peptidique comprenant deux ou plusieurs acides aminés dont les fonctions acide ou amine terminales ou ramifiées peuvent être libres ou engagées dans des groupements esters ou amides.

Dans le présent contexte, le terme "alkyle" désigne des groupes hydrocarbonés aliphatiques contenant 1 à 12 atomes de carbone, à chaîne droite ou ramifiée. On préfère les groupes alkyle inférieur, c'est-à-dire les groupes alkyle contenant 1 à 4 atomes de carbone.

Le terme "aryle" désigne les groupes aromatiques non hétérocycliques du type phényle, benzyle et les homologues supérieurs, substitués ou non, ainsi que les groupes aromatiques hétérocycliques ayant 4 à 7 atomes de carbone dans le cycle aromatique, et 1 à 4 hétéroatomes pouvant être l'oxygène, l'azote, le soufre, du type furane, pyridine, oxazole.

Le terme "cycloalkyle hétérocyclique" désigne les dérivés saturés des groupes aromatiques, hétérocycliques de type pipérazine, oxazolidine, thiazolidine, pyrrolidine.

Selon un mode de réalisation avantageux des nouveaux dérivés conformes à l'invention, le groupe

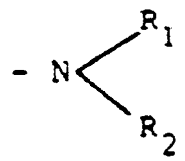

représente une amine primaire ou secondaire, linéaire, ramifiée ou cyclique,

ou bien il peut être un peptide non-soufré du type de la L-tyrosine ou de la L-histidine ;

ou encore il peut représenter un acide aminé ou un peptide soufré du type de la méthionine, de la cystéine, de la S-méthylcystéine, de la S-benzylcystéine, de la cystine , du glutathion oxydé ou non

ou bien il peut représenter un peptide de structure tyrosine-acide glutamique ou encore l'acide transurocanique.

La présente invention a également pour objet un procédé de préparation des nouveaux amides conformes à la présente invention, qui est caractérisé en ce qu'il consiste à faire réagir du chlorure d'acide para-méthoxycinnamique sur une amine ou sur une amine d'un ester d'acide amine ou d'un peptide soufré ou non soufré.

Selon un mode de mise en oeuvre du procédé de préparation des amides conformes à la présente invention, on prépare le chlorure d'acide en faisant réagir du chlorure de thionyle sur l'acide, au sein d'un solvant organique, puis on fait réagir sur le chlorure d'acide précédent, un composé à fonction amine primaire ou secondaire, au sein d'un solvant organique,en présence de triéthylamine.

La présente invention a en outre pour objet des filtres solaires dont le constituant actif est un amide de formule III ci-dessus.

L'invention a également pour objet des compositions dermo-pharmaceutiques ou cosmétologiques dont le constituant actif est un amide de formule III ci-dessus, associe à un véhicule approprié.

De telles compositions, qui peuvent avantageusement être formulées sous forme d'émulsions ou de sprays, sont notamment utiles pour le traitement des photodermatoses.

Contrairement aux produits décrits dans l'Art antérieur, les amides de l'acide p-méthoxycinnamique de formule III ci-dessus, conformes à l'invention, sont des filtres solaires qui non seulement ne se dégradent pratiquement pas ou se dégradent peu sous l'action des enzymes cutanées, mais en outre adhèrent pendant de longues durées à la surface de la peau, sans être éliminées par l'eau des bains de mer ou autres ou la transpiration, et ne pénètrent pas à travers la peau dans le circuit circulatoire sanguin.

Les dérivés objet de l'invention, possèdent des propriétés de filtres solaires intéressantes. Ils présentent un maximum d'efficacité au niveau de leur capacité d'absorption ultra-violette. Leur spectre d'absorption présente un maximum d'absorption aux environs de 310 nm, zone des UVB érythèmatogènes.

Outre les dispositions qui précèdent, l'invention comprend encore d'autres dispositions, qui ressortiront de la description qui va suivre.

L'invention sera mieux comprise à l'aide du complément de description qui va suivre, qui se réfère à des exemples de mise en oeuvre du procédé objet de la présente invention.

## EXEMPLES

### EXEMPLE I

Ester méthylique de N(méthoxy-4 cinnamoyl) L-méthionine

Dans un erlenmeyer, 1,96 g de chlorure d'acide para-méthoxycinnamique sont dissous dans 20 ml de benzène en présence de 2,8 ml de triéthylamine. 1,99 g d'ester méthylique de L-méthionine sont ajoutés progressivement. Le mélange obtenu est filtré. Après évaporation à sec sous vide (15 mm Hg) du filtrat, le résidu est repris dans de l'acide chlorhydrique N et extrait au chloroforme. La solution chloroformique est successivement lavée avec une solution aqueuse de $HKC0_3$ à 10% et avec de l'eau. Elle est ensuite recueillie, séchée sur sulfate de magnésium et évaporée. Le produit obtenu est précipité dans du cyclohexane et filtré. Après recristallisation dans un mélange éthanol 95°-eau (1:1), 2,53 g de cristaux blancs sont isolés (Rdt = 78%).

- P.F. = 95°C.
- Spectre U.V. ($CHCl_3$) : maximum 309 nm
- Spectre I.R. (KBr) : les bandes caractéristiques de la composante para-méthoxycinnamique et de la composante ester méthylique de L-méthionine sont présentes ainsi que le carbonyle amide à 1650 $cm^{-1}$.

- Spectre RMN ($CDCl_3$) : présence des signaux caractéristiques des deux composantes para-méthoxycinnamique et ester méthylique de L-méthionine.
- Constante chromatographique ; spot unique
  Rf = 0,76 (solvant A)
- P.M. : 323 (déterminé par spectrométrie de masse).

EXEMPLE II

Ester méthylique de N-(méthoxy-4 cinnamoyl) L-cystéine.

Dans un erlenmeyer, 1,96 g de chlorure d'acide para-méthoxycinnamique sont dissous dans 20 ml de benzène en présence de 2,8 ml de triéthylamine. 1,72 g d'ester méthylique de L-cystéine sont ajoutés progressivement. Le mélange obtenu est évaporé à sec sous vide. Le résidu est repris dans de l'eau, agité pendant 2h et filtré. Après recristallisation dans un mélange éthanol 95° -eau (1:1) 2,21 g de cristaux blancs sont isolés (Rdt = 78%).

- P.F. = 159° C.
- Spectre U.V. ($CHCl_3$) : maximum 314 nm.
- Spectre IR (KBr) : les bandes caractéristiques de la composante para-méthoxycinnamique et de la composante ester méthylique de L-cystéine sont présentes ainsi que le carbonyle amide à 1650 $cm^{-1}$.
- Spectre RMN ($CDCl_3$). Présence des signaux caractéristiques des deux composantes para-méthoxycinnamique et ester méthylique de L-cystéine.
- Constante chromatographique : spot unique
  Rf = 0,83 (solvant A)

EXEMPLE III

Diester méthylique de N,N'-di(méthoxy-4 cinnamoyl) L-cystine

Dans un erlenmeyer, 3,93 g de chlorure d'acide para-méthoxycinnamique sont dissous dans 20 ml de benzène en présence de 2,8 ml de triéthylamine.3,41 g de diester méthylique de cystine sont ajoutés progressivement.

La synthèse du produit est ensuite réalisée en suivant le même mode opératoire que celui utilisé pour l'exemple I. Après recristallisation dans un mélange éthanol 95° -eau (2/3:1/3) 4,46 g de cristaux blancs sont isolés (Rdt 76%).

- P.F. = 110° C.
- Spectre UV ($CHCl_3$) : maximum 309 nm
- Spectre IR (KBr) : les bandes caractéristiques de la composante para-méthoxycinnamique et de la composante diester méthylique de L-cystine sont présentes ainsi que le carbonyle amide à 1650 $cm^{-1}$.
- Spectre RMN ($CDCl_3$) : présence des signaux caractéristiques des deux composantes para-méthoxycinnamique et diester méthylique de L-cystine.
- Constante chromatographique : spot unique
  Rf : 0,71 (solvant A).
- P.M. : 588 (déterminé par spectrométrie de masse)
- Ion majoritaire 262 correspondant à la partie cystéinyl-para-méthoxycinnamique désulfurée.

EXEMPLE IV

Ester méthylique de S-méthyl N-(méthoxy-4 cinnamoyl) L-cystéine

Dans un erlenmeyer, 1,96 g de chlorure d'acide para-méthoxycinnamique sont dissous dans 20 ml de benzène en présence de 2,8 ml de triéthylamine. 1,49 g d'ester méthylique de S-méthyl L-cystéine sont ajoutés progressivement. La synthèse du produit est ensuite réalisée en suivant le même mode opératoire que celui utilisé pour l'exemple I . Après recristallisation dans un mélange éthanol 95° -eau (1:1) 2,28 g de cristaux blancs sont isolés (Rdt : 74%).

- P.F. = 98° C.
- Spectre U.V. ($CHCl_3$) : maximum 306 nm

- Spectre I.R. (KBr) : les bandes caractéristiques de la composante para-méthoxycinnamique et de la composante ester méthylique de S-méthyl L-cystéine sont présentes ainsi que le carbonyle amide à 1650 $cm^{-1}$.
- Spectre RMN ($CDCl_3$) : présence des signaux caractéristiques des deux composantes para-méthoxycinnamique et ester méthylique de S-méthyl L-cystéine.
- Constante chromatographique : spot unique
  Rf = 0,81 (solvant A).

EXEMPLE V

Ester méthylique de S-benzyl N-(méthoxy-4 cinnamoyl) L-cystéine

Dans un erlenmeyer, 1,96 g de chlorure d'acide para-méthoxycinnamique sont dissous dans 20 ml de benzène en présence de 2,8 ml de triéthylamine. 2,25 g d'ester méthylique de S-benzyl L-cystéine sont ajoutés progressivement. Le mélange obtenu est filtré. Le filtrat est acidifié en présence d'acide chlorhydrique N. La solution benzénique est recueillie et alcalinisée avec une solution aqueuse de $HKCO_3$ à 10%. Il y a précipitation de l'amide que l'on recueille par filtration. Après recristallisation dans un mélange éthanol 95° -eau (1:1), 2,61 9 de cristaux blancs sont isolés (Rdt 68%).

- P.F. = 128° C
- Spectre U.V. ($CHCl_3$) : maximum 310 nm
- Spectre I.R. (KBr) : les bandes caractéristiques de la composante para-méthoxycinnamique et de la composante ester méthylique de S-benzyl L-cystéine sont présentes ainsi que le carbonyle amide à 1650 $cm^{-1}$.
- Spectre R.M.N. ($CDCl_3$) : présence des signaux caractéristiques des deux composantes para-méthoxycinnamique et ester méthylique de S-benzyl L-cystéine.
- Constante chromatographique : spot unique
  Rf = 0,63 (solvant A).

EXEMPLE VI

Ester méthylique de N-(méthoxy-4 cinnamoyl) L-tyrosine

Dans un erlenmeyer, 1,96 g de chlorure d'acide para-méthoxycinnamique sont dissous dans 20 ml de benzène en présence de 2,8 ml de triéthylamine. 1,95 g d'ester méthylique de L-tyrosine sont ajoutés progressivement. Le mélange obtenu est évaporé à sec sous vide. Le résidu est repris dans de l'eau, agité pendant 2h et filtré. Le résidu obtenu est repris dans une solution de $HCO_3K$ à 10%, agité puis extrait au chloroforme. La solution chloroformique est recueillie, séchée sur sulfate de magnésium et évaporée. Le produit obtenu est précipité dans du cyclohexane et filtré. Après recristallisation dans un mélange éthanol 95° -eau (1:1) 2,53 9 de cristaux blancs sont isolés (Rdt : 74%).

- P.F. = 197° C.
- Spectre U.V. ($CHCl_3$) : maximum 312 nm
- Spectre I.R. (KBr) : les bandes caractéristiques de la composante para-méthoxycinnamique et de la composante ester méthylique de L-tyrosine sont présentes ainsi que le carbonyle amide à 1650 $cm^{-1}$.
- Spectre R.M.N. ($CDCl_3$) : présence des signaux caractéristiques des deux composantes para-méthoxycinnamique et ester méthylique de L-tyrosine.
- Constante chromatographique : spot unique
  Rf = 0,72 (solvant A).

EXEMPLE VII

Ester méthylique d'acide (méthoxy-4 cinnamoyl) imidazolyl-4 propénoïque-2 (amide de l'acide para-méthoxycinnamique et de l'ester méthylique de l'acide urocanique).

Dans un erlenmeyer, 1,96 g de chlorure d'acide para-méthoxycinnamique sont dissous dans 20 ml de benzène en présence de 2,8 ml de triéthylamine. 1,52 g d'ester méthylique d'acide urocanique sont ajoutés progressivement. Le mélange obtenu est filtré. Le précipité est repris dans de l'acétone anhydre et filtré. Le filtrat est évaporé à sec sous vide. Le résidu est repris dans de l'acide chlorhydrique N et extrait au

chloroforme. La solution chloroformique est successivement lavée avec une solution aqueuse de $HKCO_3$ à 10% et avec de l'eau. Elle est ensuite recueillie, séchée sur sulfate de magnésium et évaporée. Le produit obtenu est précipité dans du benzène et filtré. Après recristallisation dans un mélange éthanol 95°-eau (2/3:1/3) 2,14 g de cristaux jaune vif sont isolés (Rdt : 72%).

- P.F. = 208° C.
- Spectre U.V. (Ethanol 100°) : maximum 350 nm
- Spectre I.R. (KBr) : les bandes caractéristiques de la composante para-méthoxycinnamique et de la composante ester méthylique de l'acide urocanique sont présentes ainsi que le carbonyle amide à 1650 $cm^{-1}$.
- Spectre R.M.N. ($CDCl_3$ + DMSO - d6) : présence des signaux caractéristiques des deux composantes para-méthoxycinnamique et ester méthylique d'acide urocanique.
- Constante chromatographique : spot unique
  Rf = 0,61 (solvant A).

EXEMPLE VIII

Amide de l'acide para-méthoxycinnamique et de l'ester méthylique de glutathion oxydé.

Dans un erlenmeyer, 393 mg de chlorure d'acide para-méthoxycinnamique sont dissous dans 20 ml de benzène en présence de 2,8 ml de triéthylamine. 668 mg d'ester méthylique de glutathion oxydé sont ajoutés progressivement. La synthèse du produit est ensuite réalisée en suivant le même mode opératoire que dans l'exemple I. Après recristallisation dans un mélange éthanol 95°-eau (1:1) 501 mg de cristaux blancs sont isolés (Rdt : 51 %).

- P.F. = 124° C.
- Spectre U.V. ($CHCl_3$) : maximum 309 nm
- Spectre I.R. (KBr) : les bandes caractéristiques de la composante para-méthoxycinnamique et de la composante ester méthylique de glutathion oxydé sont présentes.
- Spectre R.M.N. ($CDCl_3$) : présence des signaux caractéristiques des deux composantes para-méthoxycinnamique et ester méthylique de glutathion oxydé.
- Constante chromatographique : Rf = 0,57 (solvant A)
- Spectrométrie de masse : ionisation chimique. Dérivatisation avec du Méthélute. m/e 536, m/e 537, m/e 538 : ions caractéristiques correspondant à la rupture du pont disulfure.

EXEMPLE IX

Amide de l'acide para-méthoxycinnamique et du méthylester de l'acide transurocanique.

En procédant comme décrit à l'exemple VIII on prépare l'amide de formule

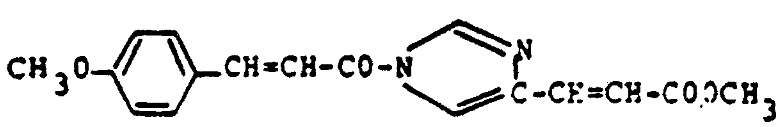

qui est capable d'absorber simultanément les UVA et les UVB.

COMPOSITION DERMO-PHARMACEUTIQUE CONTENANT L'UN DES FILTRES SOLAIRES FAISANT L'OBJET DE CETTE INVENTION.

Le filtre solaire est incorporé à la concentration 5% dans une émulsion qui peut avoir par exemple la composition suivante :

| | |
|---|---|
| Paraffine .......................................... | 12 g |
| Mono- et distéarate de PEG (Téfose 1500) ............ | 7 g |
| Acide stéarique ..................................... | 1 g |
| Alcool cétylique .................................... | 0,5 g |
| Glycéride palmitostéarique polyoxyéthyléné (Labrafil M 2130) .......... | 3 g |
| Eau ....................................... q.s.p | 100 g |

En utilisant des homogénats de peau de rat tels que décrits précédemment, on n'a pas observé de dégradation des amides selon les exemples II à IX, tandis que l'amide de l'exemple I subit une dégradation de 30 %.

Il en résulte que à l'exception de la N-(méthoxy-4-cinnamoyl)-L méthionine, qui est légèrement dégradée par les enzymes cutanés, les amides conformes à l'invention sont stables à la surface de la peau.

## Revendications

1. Amides de l'acide para-méthoxycinnamique, caractérisés en ce qu'ils répondent à la formule III ci-après :

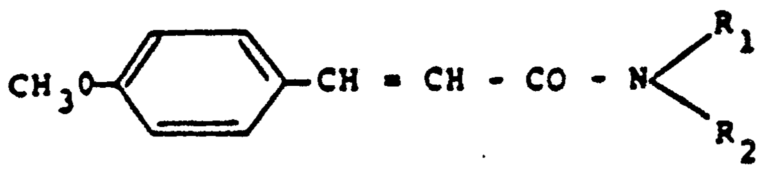

III

dans laquelle :

- les double-liaisons éthyléniques peuvent exister soit sous la forme trans, soit sous la forme cis,
- $R_1$ et $R_2$ peuvent être de l'hydrogène, un groupe alkyle, un groupe aryle, sous réserve que $R_1$ ne soit pas un atome d'hydrogène lorsque $R_2$ est un groupe alkyle ou un atome d'hydrogène
- $R_1$ et $R_2$ peuvent former avec l'atome d'azote un groupe cycloalkyle hétérocyclique substitué, ou non substitué autre que la pipéridine,
- $R_1$ ou $R_2$ peuvent aussi être un groupe :
  * de formule

  -CH-Y où X et Y
  COOX

  peuvent être un groupe alkyle ou un groupe aryle,
  * de formule

  $-CH(CH_2)_n-S-Z$
  COOX

  où X peut être un groupe alkyle ou un groupe aryle, Z peut être l'hydrogène, un groupe alkyle, un groupe aryle et où n est un nombre entier compris entre 1 et 6 mais de préférence égal à 1 ou 2,

* de formule

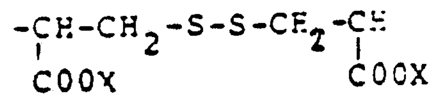

sur lequel sont fixées deux molécules de chromophores filtrantes du type acide para-méthoxycinnamique ou acide urocanique et où X peut être un groupe alkyle ou un groupe aryle,

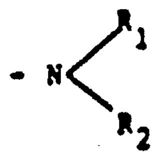

peut être un dérivé de l'acide urocanique de type

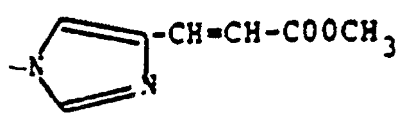

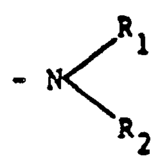

peut être une structure peptidique comprenant deux ou plusieurs acides aminés dont les fonctions acide ou amine terminales ou ramifiées peuvent être libres ou engagées dans des groupements esters ou amides.

2. Amides selon la revendication 1,caractérisés en ce que

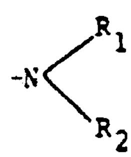

représente une amine primaire ou secondaire linéaire, ramifiée ou cyclique.

3. Amides selon la revendication 1, caractérisés en ce que

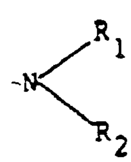

représente un acide aminé ou un peptide non soufré du type de la L-tyrosine ou de la L-histidine.

4. Amides selon la revendication 1 caractérisés en ce que

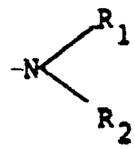

représente un acide aminé ou un peptide soufré du type de la méthionine, de la cystéine, de la S-méthyl-cystéine, de la S-benzylcystéine, de la cystine, du glutathion, du glutathion oxydé.

5. Amides selon la revendication 1, caractérisés en ce que

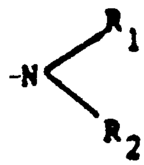

représente l'acide transurocanique.

6. Amides selon la revendication 1, caractérisés en ce que

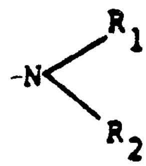

représente un peptide de structure tyrosine-acide glutamique.

7. Procédé pour l'obtention des dérivés selon l'une quelconque des revendications 1 à 4, caractérisé en ce qu'il consiste à faire réagir du chlorure d'acide para-méthoxycinnamique sur une amine ou sur une amine d'un ester d'acide aminé ou d'un peptide soufré ou non soufré.

8. Procédé selon la revendication 7, caractérisé en ce que l'on prépare le chlorure d'acide en faisant réagir du chlorure de thionyle sur l'acide,dans un solvant organique, puis l'on fait réagir sur le chlorure d'acide obtenu, un composé à fonction amine primaire ou secondaire, dans un solvant organique, en présence de triéthylamine.

9. Filtres solaires caractérisés en ce qu'ils comprennent en tant que constituant actif, au moins un amide selon la revendication 1.

10. Filtres solaires, caractérisés en ce que leur constituant actif est un amide selon l'une quelconque des revendications 4 à 6.

11. Compositions dermo-pharmaceutiques ou cosmétologiques caractérisées en ce qu'elles contiennent, à titre d'ingrédient actif, un composé selon l'une quelconque des revendications 1 à 6, en combinaison avec un véhicule approprié.

REVENDICATIONS pour l'Etat contractant suivant: AT

1. Procédé pour l'obtention des amides de l'acide para-méthoxycinnamique qui répond à la formule III ci-après :

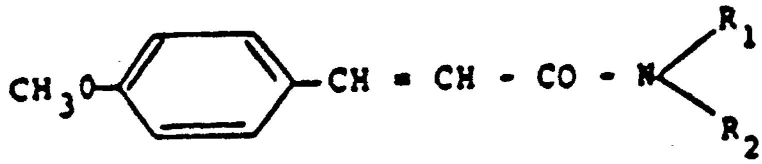

III

dans laquelle :

- les double-liaisons éthyléniques peuvent exister soit sous la forme trans, soit sous la forme cis,
- $R_1$ et $R_2$ peuvent être de l'hydrogène, un groupe alkyle, un groupe aryle, sous réserve que $R_1$ ne soit pas un atome d'hydrogène lorsque $R_2$ est un groupe alkyle ou un atome d'hydrogène,
- $R_1$ et $R_2$ peuvent former avec l'atome d'azote un groupe cycloalkyle hétérocyclique substitué, ou non substitué autre que la pipéridine,
- $R_1$ ou $R_2$ peuvent aussi être un groupe :

  * de formule

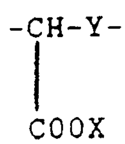

où X et Y

peuvent être un groupe alkyle ou un groupe aryle,

  * de formule

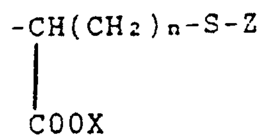

où X peut être un groupe alkyl ou un groupe aryle, Z peut être l'hydrogène, un groupe alkyle, un groupe aryle et où n est un nombre entier compris entre 1 et 6 mais de préférence égal à 1 ou 2,

  * de formule

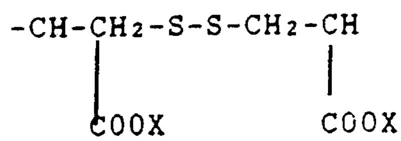

sur lequel sont fixées deux molécules de chromophores filtrantes du type acide para-méthoxycinnamique ou acide urocanique, et où X peut être un groupe alkyle ou un groupe aryle,

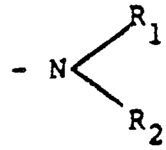

peut être un dérivé de l'acide urocanique de type

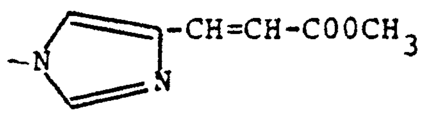

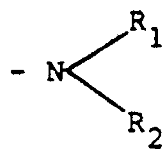

peut être une structure peptidique comprenant deux ou plusieurs acides aminés dont les fonctions acide ou amine terminales ou ramifiées peuvent être libres ou engagées dans des groupements esters ou amides, caractérisé en ce qu'il consiste à faire réagir du chlorure d'acide para-méthoxycinnamique sur une amine ou sur une amine d'un ester d'acide aminé ou d'un peptide soufré ou non soufré.

**2.** Procédé selon la Revendication 1, caractérisé en ce que l'on prépare le chlorure d'acide en faisant réagir du chlorure de thionyle sur l'acide, dans un solvant organique, puis l'on fait réagir sur le chlorure d'acide obtenu, un composé à fonction amine primaire ou secondaire, dans un solvant organique, en présence de triéthylamine.

**3.** Procédé selon l'une quelconque des Revendications 1 et 2, caractérisé en ce que

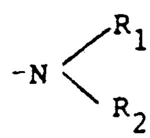

représente une amine primaire ou secondaire linéaire, ramifiée ou cyclique.

**4.** Procédé selon l'une quelconque des Revendications 1 et 2,caractérisé en ce que

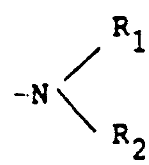

représente un acide aminé ou un peptide non soufré du type de la L-tyrosine ou de la L-histidine.

**5.** Procédé selon l'une quelconque des Revendications 1 et 2, caractérisé en ce que

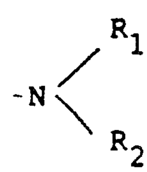

représente un acide aminé ou un peptide soufré du type de la méthionine, de la cystéine, de la S-méthyl-cystéine, de la S-benzylcystéine, de la cystine, du glutathion, du glutathion oxydé.

6. Procédé selon l'une quelconque des Revendications 1 et 2, caractérisé en ce que

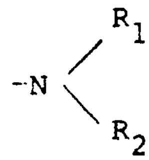

représente un peptide de structure tyrosine-acide glutamique.

## Claims

1. A paramethoxycinnamic acid amide, characterized in that it has formula III below:

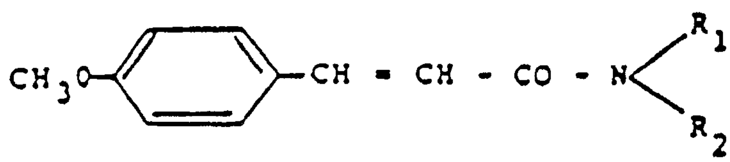

III

in which:

- the ethylenic double bonds can exist either in the trans form or in the cis form, and
- $R_1$ and $R_2$ can be hydrogen, an alkyl group or an aryl group, with the proviso that $R_1$ is not a hydrogen atom when $R_2$ is an alkyl group or a hydrogen atom, or
- $R_1$ and $R_2$ can form, with the nitrogen atom, a substituted or unsubstituted heterocyclic cycloalkyl group other than piperidine, or
- $R_1$ or $R_2$ can be a group
  * of the formula

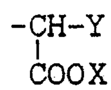

in which X and Y can be an alkyl group or an aryl group,

* of the formula

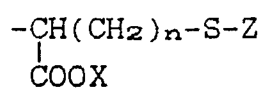

in which X can be an alkyl group or an aryl group, Z can be hydrogen, an alkyl group or an aryl group and n is an integer between 1 and 6, but preferably equal to 1 or 2, or

* of the formula

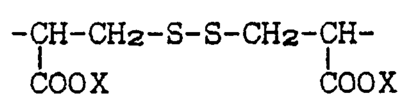

to which two filtering chromophore molecules of the paramethoxycinnamic acid or urocanic acid type are attached and in which X can be an alkyl group or an aryl group, or

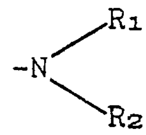

can be a urocanic acid derivative of the type

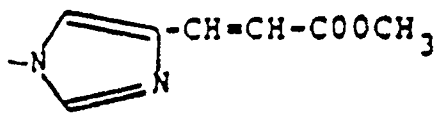

or

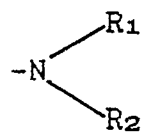

can be a peptide structure comprising two or more amino acids whose terminal or branched acid or amine groups can be free or part of ester or amide groups.

**2.** An amide according to Claim 1, characterized in that

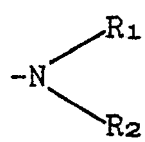

is a linear, branched or cyclic primary or secondary amine.

**3.** An amide according to Claim 1, characterized in that

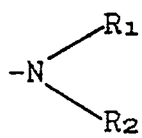

is an amino acid or a peptide not containing sulphur, of the L-tyrosine or L-histidine type.

**4.** An amide according to Claim 1, characterized in that

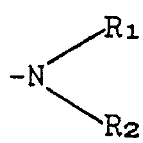

is an amino acid or a peptide containing sulphur, of the methionine, cysteine, S-methylcysteine, S-benzylcysteine, cystine, glutathion or oxidized glutathion type.

**5.** An amide according to Claim 1, characterized in that

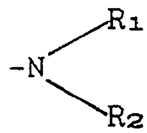

is trans-urocanic acid.

6. An amide according to Claim 1, characterized in that

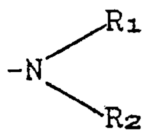

is a peptide of tyrosine-glutamic acid structure.

7. A method of obtaining the derivatives according to any one of Claims 1 to 4, characterized in that it consists in reacting paramethoxycinnamoyl chloride with an amine or with an amine of an amino acid ester or of a peptide which may or may not contain sulphur.

8. A method according to Claim 7, characterized in that the acid chloride is prepared by reacting thionyl chloride with the acid in an organic solvent, after which a compound containing a primary or secondary amine group is reacted with the resulting acid chloride in an organic solvent, in the presence of triethylamine.

9. A solar filter, characterized in that it comprises at least one amide according to Claim 1 as the active constituent.

10. A solar filter, characterized in that its active constituent is an amide according to any one of Claims 4 to 6.

11. A dermopharmaceutical or cosmetological composition, characterized in that it contains, as the active ingredient, a compound according to any one of Claims 1 to 6 in combination with an appropriate vehicle.

CLAIMS for the following Contracting State : AT

1. A method of obtaining the paramethoxycinnamic acid amides of formula III below:

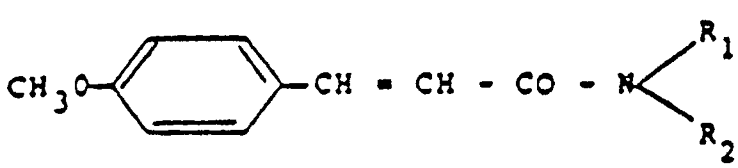

III

in which:

- the ethylenic double bonds can exist either in the trans form or in the cis form, and
- $R_1$ and $R_2$ can be hydrogen, an alkyl group or an aryl group, with the proviso that $R_1$ is not a hydrogen atom when $R_2$ is an alkyl group or a hydrogen atom, or
- $R_1$ and $R_2$ can form, with the nitrogen atom, a substituted or unsubstituted heterocyclic cycloalkyl group other than piperidine, or
- $R_1$ or $R_2$ can be a group
  * of the formula

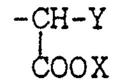

in which X and Y can be an alkyl group or an aryl group,
* of the formula

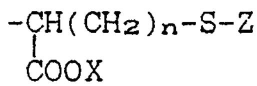

in which X can be an alkyl group or an aryl group, Z can be hydrogen, an alkyl group or an aryl group and n is an integer between 1 and 6, but preferably equal to 1 or 2, or
* of the formula

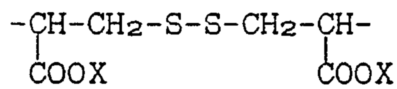

to which two filtering chromophore molecules of the paramethoxycinnamic acid or urocanic acid type are attached and in which X can be an alkyl group or an aryl group, or

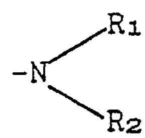

can be a urocanic acid derivative of the type

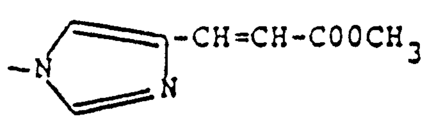

or

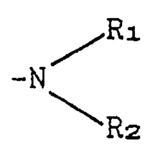

can be a peptide structure comprising two or more amino acids whose terminal or branched acid or amine groups can be free or part of ester or amide groups, characterized in that it consists in reacting paramethoxycinnamoyl chloride with an amine or with an amine of an amino acid ester or of a peptide which may or may not contain sulphur.

2. A method according to Claim 1, characterized in that the acid chloride is prepared by reacting thionyl chloride with the acid in an organic solvent, after which a compound containing a primary or secondary amine group is reacted with the resulting acid chloride in an organic solvent, in the presence of triethylamine.

3. A method according to Claim 1 or Claim 2, characterized in that

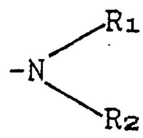

is a linear, branched or cyclic primary or secondary amine.

4. A method according to Claim 1 or Claim 2, characterized in that

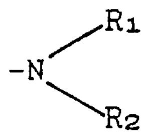

is an amino acid or a peptide not containing sulphur, of the L-tyrosine or L-histidine type.

5. A method according to Claim 1 or Claim 2, characterized in that

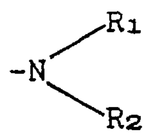

is an amino acid or a peptide containing sulphur, of the methionine, cysteine, S-methylcysteine, S-benzylcysteine, cystine, glutathion or oxidized glutathion type.

6. A method according to Claim 1 or Claim 2, characterized in that

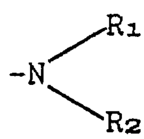

is a peptide of tyrosine-glutamic acid structure.

## Ansprüche

1. Paramethoxyzimtsäureamide, dadurch **gekennzeichnet**, daß sie der folgenden Formel III

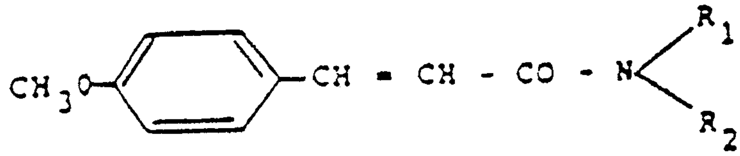

III

entsprechen, worin:

- die ethylenischen Doppelbindungen in Trans- oder in Cis-Form vorliegen können,
- $R_1$ und $R_2$ Wasserstoff, eine Alkylgruppe, eine Arylgruppe sein können, mit der Maßgabe, daß $R_1$ kein Wasserstoffatom ist, wenn $R_2$ eine Alkylgruppe oder ein Wasserstoffatom ist,
- $R_1$ und $R_2$ mit dem Stickstoffatom eine substituierte oder nichtsubstituierte heterocyclische Cycloalkylgruppe, außer Piperidin, bilden können,
- $R_1$ oder $R_2$ auch eine Gruppe:

* der Formel

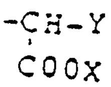

worin X und Y eine Alkyl- oder eine Arylgruppe sein können,
* der Formel

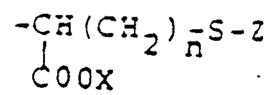

worin X eine Alkyl- oder eine Arylgruppe sein kann, Z Wasserstoff, eine Alkylgruppe, eine Arylgruppe sein kann und worin n eine ganze Zahl zwischen 1 und 6, aber bevorzugt 1 oder 2 ist,
* der Formel

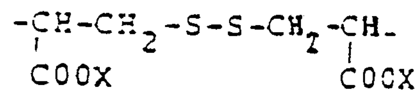

an welche zwei Moleküle Filterchromophore vom Typ Parамethoxyzimtsäure oder Urocaninsäure gebunden sind und worin X eine Alkyl- oder eine Arylgruppe sein kann, sein kann,

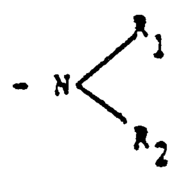

ein Urocaninsäurederivat vom Typ

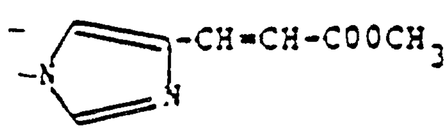

sein kann,

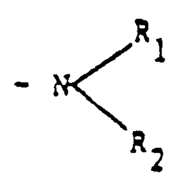

eine Peptidstruktur sein kann mit 2 oder mehreren Aminosäuren, deren terminale oder verzweigte Säure- oder Aminfunktionen frei oder in Ester- oder Amidgruppen gebunden sein können.

**2.** Amide nach Anspruch 1, dadurch **gekennzeichnet**, daß

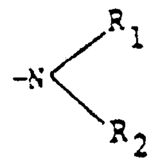

ein lineares, verzweigtes oder cyclisches primäres oder sekundäres Amin bedeutet.

3. Amide nach Anspruch 1, dadurch **gekennzeichnet,** daß

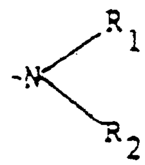

eine Aminosäure oder ein nicht schwefelhaltiges Peptid vom Typ L-Tyrosin oder L-Histidin bedeutet.

4. Amide nach Anspruch 1, dadurch **gekennzeichnet,** daß

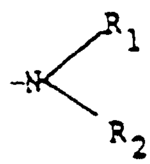

eine Aminosäure oder ein schwefelhaltiges Peptid vom Typ Methionin, Cystein, S-Methylcystein, S-Benzylcystein, Cystin, Glutathion, Glutathionoxid bedeutet.

5. Amide nach Anspruch 1, dadurch **gekennzeichnet,** daß

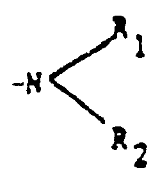

Transurocaninsäure bedeutet.

6. Amide nach Anspruch 1, dadurch **gekennzeichnet,** daß

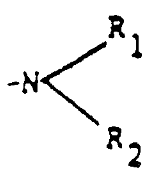

ein Peptid der Struktur Tyrosin-Glutaminsäure bedeutet.

7. Verfahren zur Herstellung der Derivate nach einem der Ansprüche 1 bis 4, dadurch **gekennzeichnet,** daß man Paramethoxyzimtsäurechlorid mit einem Amin oder einem Aminosäureesteramid entweder eines schwefelhaltigen oder nicht schwefelhaltigen Peptids zur Reaktion bringt.

8. Verfahren nach Anspruch 7, dadurch **gekennzeichnet**, daß man das Säurechlorid durch Umsetzung von Thionylchlorid mit der Säure in einem organischen Lösungsmittel herstellt, anschließend das erhaltene Säurechlorid mit einer Verbindung mit einer primären oder sekundären Aminfunktion in einem organischen Lösungsmittel in Gegenwart von Triethylamin umsetzt.

9. Sonnenfilter, dadurch **gekennzeichnet**, daß sie als wirksamen Bestandteil mindestens ein Amid nach Anspruch 1 umfassen.

10. Sonnenfilter, dadurch **gekennzeichnet**, daß ihr wirksamer Bestandteil ein Amid nach einem der Ansprüche 4 bis 6 ist.

11. Dermopharmazeutische oder kosmetologische Präparate, dadurch **gekennzeichnet**, daß sie als wirksamen Inhaltsstoff eine Verbindung nach einem der Ansprüche 1 bis 6 zusammen mit einem geeigneten Träger enthalten.

PATENTANSPRÜCHE für folgenden Vertragsstaat : AT

1. Verfahren zur Herstellung von Paramethoxyzimtsäureamiden, die der folgenden Formel III

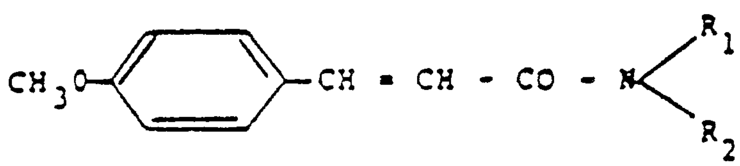

III

entsprechen, worin:
- die ethylenischen Doppelbindungen in Trans- oder in Cis-Form vorliegen können,
- $R_1$ und $R_2$ Wasserstoff, eine Alkylgruppe, eine Arylgruppe sein können, mit der Maßgabe, daß $R_1$ kein Wasserstoffatom ist, wenn $R_2$ eine Alkylgruppe oder ein Wasserstoffatom ist,
- $R_1$ und $R_2$ mit dem Stickstoffatom eine substituierte oder nichtsubstituierte heterocyclische Cycloalkylgruppe, außer Piperidin, bilden können,
- $R_1$ oder $R_2$ auch eine Gruppe:
  * der Formel

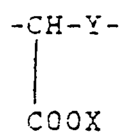

  worin X und Y eine Alkyl- oder eine Arylgruppe sein können,
  * der Formel

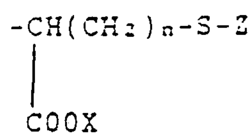

  worin X eine Alkyl- oder eine Arylgruppe sein kann, Z Wasserstoff, eine Alkylgruppe, eine Arylgruppe sein kann und worin n eine ganze Zahl zwischen 1 und 6, aber bevorzugt 1 oder 2 ist,
  * der Formel

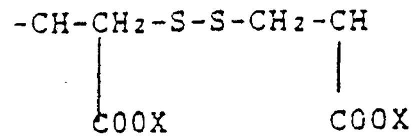

an welche zwei Moleküle Filterchromophore vom Typ Paramethoxyzimtsäure oder Urocaninsäure gebunden sind und worin X eine Alkyl- oder eine Arylgruppe sein kann, sein kann,

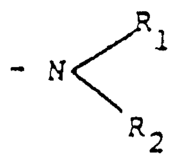

ein Urocaninsäurederivat vom Typ

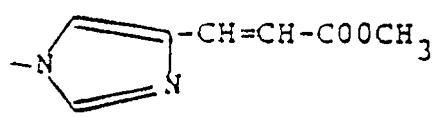

sein kann,

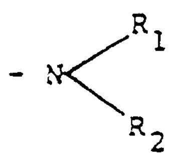

eine Peptidstruktur sein kann mit 2 oder mehreren Aminosäuren, deren terminale oder verzweigte Säure- oder Aminfunktionen frei oder in Ester- oder Amidgruppen gebunden sein können, dadurch **gekennzeichnet**, daß man ein Paramethoxyzimtsäurechlorid mit einem Amin oder einem Aminosäureesteramid entweder eines schwefelhaltigen oder nicht schwefelhaltigen Peptids zur Umsetzung bringt.

2. Verfahren nach Anspruch 1, dadurch **gekennzeichnet**, daß man das Säurechlorid durch Umsetzung von Thionylchlorid mit der Säure in einem organischen Lösungsmittel herstellt, anschließend auf das erhaltene Säurechlorid eine Verbindung mit einer primären oder sekundären Aminfunktion in einem organischen Lösungsmittel in Gegenwart von Triethylamin einwirken läßt.

3. Verfahren nach einem der Ansprüche 1 und 2, dadurch **gekennzeichnet**, daß

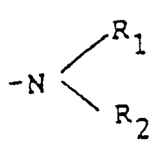

ein lineares, verzweigtes oder cyclisches primäres oder sekundäres Amin bedeutet.

4. Verfahren nach einem der Ansprüche 1 und 2, dadurch **gekennzeichnet**, daß

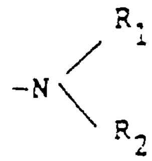

eine Aminosäure oder ein nicht schwefelhaltiges Peptid vom Typ L-Tyrosin oder L-Histidin bedeutet.

5. Verfahren nach einem der Ansprüche 1 und 2, dadurch **gekennzeichnet**, daß

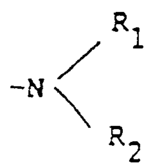

eine Aminosäure oder ein schwefelhaltiges Peptid vom Typ Methionin, Cystein, S-Methylcystein, S-Benzylcystein, Cystin, Glutathion, Glutathionoxid bedeutet.

6. Verfahren nach einem der Ansprüche 1 und 2, dadurch **gekennzeichnet**, daß

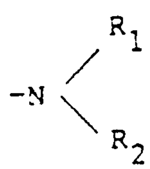

ein Peptid der Struktur Tyrosin-Glutaminsäure bedeutet.